# EUROPEAN PATENT APPLICATION

(11) **EP 4 685 222 A1**
(43) Date of publication of application: **28.01.2026**
(21) Application number: 24775004.5
(22) Date of filing: 22.03.2024
(51) Int. Cl.: C12M 1/04, B01D 53/04, C07G 99/00, C12N 1/20

(54) **SYSTEM FOR PRODUCING ORGANIC SUBSTANCE, DEVICE FOR PRODUCING ORGANIC SUBSTANCE, AND METHOD FOR PRODUCING ORGANIC SUBSTANCE**

(30) Priority: 23.03.2023 JP 2023047370
(71) Applicant: Sekisui Chemical Co., Ltd., Osaka-shi, Osaka 530-8565 (JP)
(72) Inventor: NOMURA, Shun, Tokyo 105-8566 (JP); KAWAI, Hiroshi, Tokyo 105-8566 (JP); HIGASHI, Eiji, Tokyo 105-8566 (JP)
(74) Representative: Ter Meer Steinmeister & Partner
(86) International application number: PCT/JP2024/011496
(87) International publication number: WO 2024/195871

(57) **Abstract**

[Problem]

To provide an organic substance production system and the like capable of reducing thermal energy used in pretreatment and efficiently removing impurities from a raw material gas.

[Methods]

An organic substance production system comprises: a gas generation unit configured to generate raw material gas including at least carbon monoxide, carbon dioxide, and monocyclic aromatic compounds; first and second separation sections including first adsorbent in a first housing section and second adsorbent in a second housing section, the first adsorbent capable of adsorbing at least carbon dioxide and the second adsorbent capable of adsorbing at least monocyclic aromatic compounds; a depressurization mechanism configured to depressurize the inside of the first housing section and the second housing section, first and second separation sections configured to separate the carbon dioxide and the monocyclic aromatic compounds by depressurization; and a bioreactor configured to receive raw material gas from which the carbon dioxide and the monocyclic aromatic compounds have been separated and to produce an organic substance from carbon monoxide included in the supplied raw material gas by action of gas-assimilating bacteria.

## Description

### TECHNICAL FIELD

The present disclosure relates to an organic substance production system, organic substance production apparatus and organic substance production method. BACKGROUND ART

In recent years, global environmental issues have arisen, including concerns about depletion of fossil fuel resources due to mass consumption of oils, alcohols, and similar materials produced from petroleum, and an increase of carbon dioxide in the atmosphere. To address these issues, methods for producing organic substances using raw materials other than petroleum have attracted attention, for example, a method of producing bioethanol by a sugar-fermentation process using edible feedstocks such as corn.

However, the sugar-fermentation process using such edible feedstocks may use limited farmland for non-food production and thereby risk causing rises in food prices. Accordingly, methods have been studied for producing organic substances previously produced from petroleum-using non-edible feedstocks that used to be discarded.

For example, Patent Literature 1 discloses a method for producing an organic substance by microbial fermentation of synthesis gas including at least carbon monoxide. The document also discloses performing a pretreatment on the synthesis gas to remove impurities included in the synthesis gas prior to microbial fermentation.

### PRIOR ART DOCUMENTS

### Patent document

[Patent Document 1] Japanese Laid-Open Patent Publication No. 2019-188730 (Re-publication)

### SUMMARY

### Problems to be Solved by Invention

However, according to the inventors' studies, when a separation device of a temperature swing adsorption (TSA) method is used in the pretreatment, a large amount of thermal energy is required.

In view of the foregoing, the present disclosure aims to provide, among other things, an organic substance production system capable of reducing thermal energy used in pretreatment and efficiently removing impurities from a raw material gas. Methods for Solving Problems

According to one aspect, an organic substance production system is provided. The production system includes: a gas generation unit that generates a raw material gas including at least carbon monoxide, carbon dioxide, and monocyclic aromatic compounds; a first separation section having a first adsorbent capable of adsorbing at least the carbon dioxide in the raw material gas and a first housing section in which the first adsorbent is disposed, the first separation section being configured to separate the carbon dioxide from the first adsorbent by depressurizing an inside of the first housing section; a second separation section having a second adsorbent capable of adsorbing at least the monocyclic aromatic compounds in the raw material gas and a second housing section in which the second adsorbent is disposed, the second separation section being configured to separate the monocyclic aromatic compounds from the second adsorbent by depressurizing an inside of the second housing section; a depressurization mechanism capable of depressurizing the inside of the first housing section and the inside of the second housing section; and a bioreactor that receives the raw material gas from which the carbon dioxide and the monocyclic aromatic compounds have been separated and produces an organic substance from the carbon monoxide included in the supplied raw material gas by an action of gas-utilizing bacteria.

According to the foregoing aspect, impurities can be efficiently removed from the raw material gas while reducing thermal energy used.

### BRIEF DESCRIPTION OF DRAWINGS

FIG. 1 is a schematic diagram showing a configuration of an organic substance production system according to a first embodiment.
FIG. 2 is a schematic diagram showing a configuration of a separation device in the organic substance production system according to the first embodiment.
FIG. 3 is a schematic diagram showing a configuration of an organic substance production system according to a second embodiment.
FIG. 4 is a schematic diagram showing a configuration of an organic substance production system according to a third embodiment.
FIG. 5 is a schematic diagram showing a configuration of an organic substance production system according to a fourth embodiment.
FIG. 6 is a schematic diagram showing a configuration of a separation device in the organic substance production system according to the fourth embodiment.

### DETAILED DESCRIPTION

The following describes an organic-substance production system, an organic-substance production apparatus, and an organic-substance production method in detail based on preferred embodiments shown in the accompanying drawings.

### <First Embodiment>

First, a description is given of an organic substance production system according to the first embodiment.

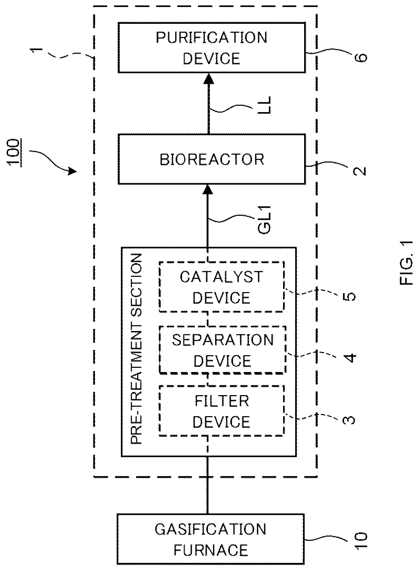

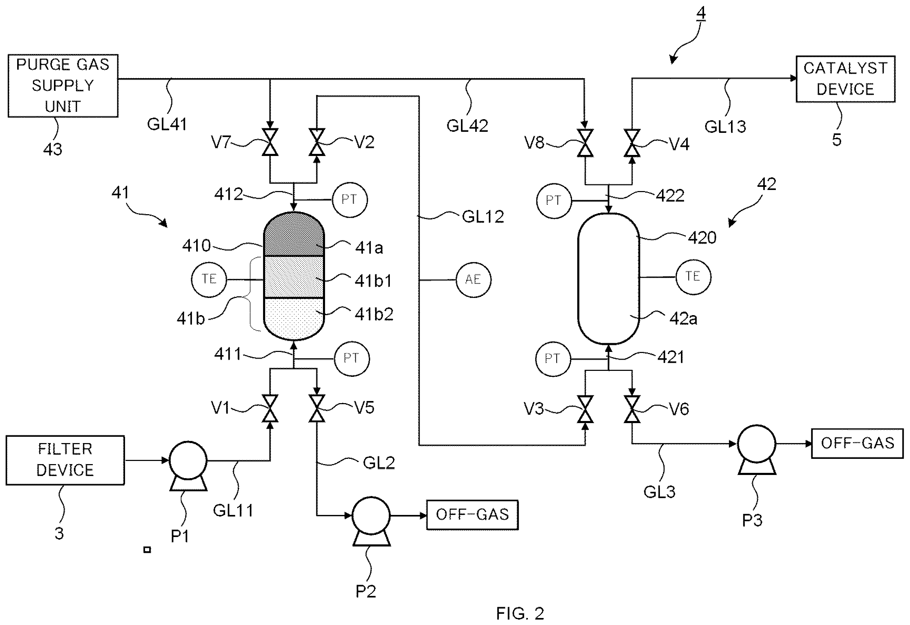

FIG. 1 is a schematic diagram showing a configuration of an organic substance production system according to a first embodiment. FIG. 2 is a schematic diagram showing a configuration of a separation device in the organic substance production system according to the first embodiment.

An organic substance production system 100 (also simply referred to as "production system 100") includes a gasification furnace (gas generation unit) 10 and an organic substance production apparatus 1 connected to the gasification furnace 10 (also simply referred to as "production apparatus 1"). The production apparatus 1 is used in connection with the gasification furnace (gas generation unit) 10.

In this specification, for a flow direction of a raw material gas and a liquid, an upstream side is simply referred to as "upstream," and a downstream side as "downstream."

In the present embodiment, examples of the gasification furnace 10 include a combustion furnace, a blast furnace, a converter, an electric furnace, and a shaft furnace. The gas generation unit may also be a COₓ emission source at least one site selected from a paper mill, cement plant, thermal power plant, refinery, ethylene cracker, oil refinery, or chemical plant, in addition to the gasification furnace 10.

Each furnace generates (produces) an exhaust gas (raw material gas) including at least carbon monoxide, carbon dioxide, and monocyclic aromatic compounds during combustion, melting, or refining of contents.

In the case of a combustion furnace (incinerator) at a waste incineration plant, examples of the contents (waste) include plastic waste, food waste, municipal solid waste (MSW), industrial waste, waste tires, biomass waste, household waste (bedding, paper), and building materials. The waste may include one type alone or two or more types.

The exhaust gas may further include, in addition to carbon monoxide, carbon dioxide, and monocyclic aromatic compounds, other gas components such as nitrogen, hydrogen, oxygen, moisture, and methane. The exhaust gas may further include, as other components, soot, tar, nitrogen compounds, sulfur compounds, phosphorus compounds, halogen compounds, cyanide compounds, and polycyclic aromatic compounds.

The exhaust gas may be produced as a gas containing 10 vol% or more of carbon monoxide by subjecting the contents (carbon source) to heat treatment causing incomplete combustion (commonly referred to as gasification), that is, partial oxidation of the carbon source.

By using the exhaust gas, carbon dioxide conventionally emitted into the atmosphere can be effectively utilized, which reduces environmental load. From the standpoint of carbon cycling, exhaust gas generated in combustion furnaces or smelting facilities is preferred.

The production apparatus 1 is connected to the gasification furnace 10. The production apparatus 1 includes a bioreactor 2, a purification device 6, a gas line GL1 that connects the gasification furnace 10 and the bioreactor 2, and a liquid line LL that connects the bioreactor 2 and the purification device 6.

In the bioreactor 2, an organic substance is produced from the supplied exhaust gas (raw material gas) by an action of gas-utilizing bacteria (microbial fermentation). Specifically, in the bioreactor 2, an organic-substance-containing solution that contains an organic substance is obtained.

The gas-utilizing bacteria include both eubacteria and archaea.

Examples of the eubacteria include bacteria of the genera Clostridium, Moorella, Acetobacterium, Carboxydocella, Rhodopseudomonas, Eubacterium, Butyribacterium, Oligotropha, Bradyrhizobium, and aerobic hydrogen-oxidizing bacteria of the genus Ralstonia.

Examples of the archaea include bacteria of the genera Methanobacterium, Methanobrevibacter, Methanocalculus, Methanococcus, Methanosarcina, Methanosphaera, Methanothermobacter, Methanothrix, Methanoculleus, Methanofollis, Methanogenium, Methanospirillum, Methanosaeta, Thermococcus, Thermofilum, and Archaeoglobus.

Among such gas-utilizing bacteria, bacteria having a high ability to produce a target organic substance are selected for use. For example, as gas-utilizing bacteria having high ethanol-producing ability, bacteria such as Clostridium autoethanogenum, Clostridium ljungdahlii, Clostridium aceticum, Clostridium carboxidivorans, Moorella thermoacetica, and Acetobacterium woodii can be mentioned.

A medium (culture liquid) used when culturing the gas-utilizing bacteria is not particularly limited as long as the composition is appropriate for the type. For example, when bacteria of the genus Clostridium are used as the gas-utilizing bacteria, paragraphs [0091] of WO 2017/117309 and [0097]-[0098] of US 2017/0260552 may be referred to for the medium.

Examples of the bioreactor 2 include a type of culture reactor that agitates the culture liquid with a stirring blade, a type of culture reactor that agitates the culture liquid by circulating the culture liquid itself, and a type of culture reactor that agitates the culture liquid by a water flow accompanying a bubble flow arising from aeration by the supplied exhaust gas.

A pretreatment section that removes impurities from the exhaust gas is provided at an intermediate position of the gas line GL1.

The pretreatment section includes, in order from the gasification furnace 10 side (upstream), a filter device 3, a separation device 4, and a catalyst device 5.

The filter device 3 is used to remove tar, soot, and fine particles smaller than soot. The filter can be configured with, for example, a bag filter.

The catalyst device 5 is used mainly to remove oxygen and acetylene.

As an oxygen-removal catalyst, particles of metals such as copper (Cu), platinum (Pt), and nickel (Ni) can be used. The oxygen-removal catalyst is preferably heated to 150°C to 400 °C.

As an acetylene-removal catalyst, particles of noble metals such as palladium (Pd) and platinum (Pt) can be used.

Removal of acetylene prior to deoxygenation provides an advantage of suitably preventing or reducing adverse effects of acetylene on the oxygen-removal catalyst.

As shown in FIG. 2, the separation device 4 includes a first separation section 41 and a second separation section 42.

The first separation section 41 and the second separation section 42 each include a first vessel (first housing section) 410 and a second vessel (second housing section) 420, respectively, provided at an intermediate position of the gas line GL1 in this order from upstream. The gas line GL1 is configured with a gas line GL11, a gas line GL12, and a gas line GL13. The numbers "first" and "second" are only for distinction and do not limit an arrangement order. Accordingly, an adsorbent for carbon dioxide may be disposed downstream of an adsorbent for monocyclic aromatic compounds. The order of disposing the adsorbents is more preferably such that the adsorbent for carbon dioxide is disposed downstream of the adsorbent for monocyclic aromatic compounds. With the foregoing order, a target substance can be efficiently adsorbed.

The gas line GL11 connects the filter device 3 and a lower port 411 of the first vessel 410. An air supply pump (compressor) P1 and a valve V1 are provided in this order from upstream at an intermediate position of gas line GL11.

The gas line GL12 connects an upper port 412 of the first vessel 410 and a lower port 421 of the second vessel 420. A valve V2 and a valve V3 are provided in this order from upstream at an intermediate position of gas line GL12.

The gas line GL13 connects an upper port 422 of the second vessel 420 and the catalyst device 5. A valve V4 is provided at an intermediate position of the gas line GL13.

With the foregoing configuration, the exhaust gas (raw material gas) can be passed sequentially through the first vessel 410 and the second vessel 420 via the gas line GL1.

A gas line GL2 is connected to the lower port 411 of the first vessel 410. A valve V5 and a first depressurization pump (first depressurization section) P2 are provided in this order from the first vessel 410 side at an intermediate position of gas line GL2. The first depressurization pump P2 is connected to the lower port 411 (a side to which the exhaust gas is supplied) of the first vessel 410. Herein, "depressurization" refers to a state in which, when comparing before and after depressurization, a calculated pressure after depressurization is lower by 20 kPa or more than a pressure before depressurization. The pressure reduction may be 30 kPa or more and 400 kPa or less in calculation. More preferably, the pressure reduction is 60 kPa or more and 200 kPa or less in calculation. If the pressure reduction exceeds 400 kPa, depressurization may take too long. Conversely, if the pressure reduction is less than 20 kPa, a sufficient depressurization effect may not be obtained.

A gas line GL3 is connected to the lower port 421 of the second vessel 420. A valve V6 and a second depressurization pump (second depressurization section) P3 are provided in this order from the second vessel 420 side at an intermediate position of gas line GL3. The second depressurization pump P3 is connected to the lower port 421 (a side to which the exhaust gas is supplied) of the second vessel 420.

With the foregoing configuration, the inside of the first vessel 410 can be depressurized via the gas line GL2 by an action of the first depressurization pump P2, and the inside of the second vessel 420 can be depressurized via the gas line GL3 by an action of the second depressurization pump P3. In the present embodiment, a depressurization mechanism capable of reducing the pressure in the first vessel (first housing section) 410 and the second vessel (second housing section) 420 is configured with the first depressurization pump (first depressurization section) P2 and the second depressurization pump (second depressurization section) P3.

The first vessel 410 accommodates a first adsorbent 41a disposed therein and capable of adsorbing at least carbon dioxide in the exhaust gas. Separation of carbon dioxide is achieved by depressurizing the first vessel 410 with the first depressurization pump P2, thereby desorbing carbon dioxide from the first adsorbent 41a. The first separation section 41 is primarily configured with the first vessel 410, the first adsorbent 41a, and the first depressurization pump P2.

The first adsorbent 41a may be composed of one or more materials selected from zeolite, bentonite, sericite, perlite, coral reef rock, vermiculite, silica gel, molecular sieves, activated carbon, and metal-organic frameworks (MOFs). Among these, the first adsorbent 41a is preferably constituted by zeolite. This is because zeolite particularly excels in adsorption and desorption of carbon dioxide.

The average particle diameter of the first adsorbent 41a is preferably 0.5 mm to 10 mm, more preferably 0.8 mm to 8 mm, and even more preferably 1 mm to 6 mm. Within these ranges, a sufficiently large contact area between the first adsorbent 41a and the exhaust gas can be obtained. The shape is not limited to a spherical shape; for example, a cylindrical shape may be used.

In this specification, "average particle diameter" means the average of the particle diameters of 200 arbitrarily selected particles within a single field of view observed under an electron microscope. Here, "particle diameter" means the maximum distance between two points on the outline of a particle.

The BET specific surface area of the first adsorbent 41a is preferably 50 m²/g to 1000 m²/g, more preferably 200 m²/g to 800 m²/g, and even more preferably 300 m²/g to 700 m²/g. Within these ranges, a sufficiently large contact area between the first adsorbent 41a and the exhaust gas can be obtained.

In this specification, the BET specific surface area refers to the value measured with a BET surface-area analyzer in accordance with the one-point BET method specified in JIS R 1626:1996 (Measuring methods for the specific surface area of fine ceramic powders by gas adsorption using the BET method).

The average pore size of the first adsorbent 41a is preferably 1 Å to 20 Å, more preferably 3 Å to 18 Å, and even more preferably 5 Å to 15 Å. Within these ranges, a sufficiently large contact area between the first adsorbent 41a and the exhaust gas can be obtained.

In this specification, "average pore size" refers to the value measured by a nitrogen adsorption method or a positron annihilation method.

When the exhaust gas further includes moisture, sulfur compounds, polycyclic aromatic compounds, and tar, the first separation section 41 is preferably configured to adsorb and separate the moisture, the sulfur compounds, the polycyclic aromatic compounds, and the tar. This prevents adverse effects on the growth of gas-assimilating bacteria.

Examples of sulfur compounds include hydrogen sulfide, carbonyl sulfide, mercuric sulfide, sulfur dioxide, sulfur trioxide, sulfur hexafluoride, sulfur dichloride, and sodium thiosulfate. Examples of polycyclic aromatic compounds include naphthalene, anthracene, phenanthrene, tetracene, pentacene, chrysene, and benzo[a]pyrene.

In the present embodiment, the first vessel 410 further accommodates a protective agent 41b disposed therein and capable of adsorbing the moisture, the sulfur compounds, the polycyclic aromatic compounds, and the tar, thereby protecting the first adsorbent 41a. Accommodating the protective agent 41b in the first vessel 410 provides, in addition to the effect described above, the effect of preventing or suppressing time-dependent deterioration in the performance of the first adsorbent 41a. The protective agent 41b may be accommodated in a vessel other than the first vessel 410, which accommodates the first adsorbent 41a. In this case, the other vessel is preferably disposed upstream of the first vessel 410.

The protective agent 41b preferably includes a first protective agent 41b1 capable of adsorbing moisture and sulfur compounds, and a second protective agent 41b2 capable of adsorbing polycyclic aromatic compounds and tar. In this case, by using, as the first protective agent 41b1 and the second protective agent 41b2, protective agents that differ from each other in at least one of average particle diameter, BET specific surface area, average pore size, density, temperature resistance, or shape, the adsorption and desorption of moisture, sulfur compounds, polycyclic aromatic compounds, and tar can be suitably enhanced.

Specifically, as shown in FIG. 2, the first vessel 410 accommodates, in the order from the lower port 411 side, which is the side to which the exhaust gas is supplied, the second protective agent 41b2, the first protective agent 41b1, and the first adsorbent 41a, each disposed therein. According to this configuration, before the exhaust gas contacts the first adsorbent 41a, the protective agent 41b adsorbs moisture, sulfur compounds, polycyclic aromatic compounds, and tar, thereby more reliably preventing or suppressing time-dependent deterioration in the activity of the first adsorbent 41a.

The first protective agent 41b1 and the second protective agent 41b2 may each be composed of the same materials as those listed for the first adsorbent 41a. Preferably, the first protective agent 41b1 is composed of zeolite, and the second protective agent 41b2 is composed of activated carbon. In this case, the first protective agent 41b1 exhibits excellent adsorption of moisture and sulfur compounds, and the second protective agent 41b2 exhibits excellent adsorption of polycyclic aromatic compounds and tar.

In this case, the first adsorbent 41a is preferably composed of zeolite having a larger pore size than that of the zeolite used for the first protective agent 41b1. With the substances that adversely affect the first adsorbent 41a removed, the first adsorbent 41a is capable of sufficiently and reliably adsorbing and desorbing carbon dioxide.

The second vessel 420 accommodates a second adsorbent 42a disposed therein and capable of adsorbing at least monocyclic aromatic compounds in exhaust gas from which carbon dioxide has been separated by the first separation section 41. The second adsorbent 42a enables separation of the monocyclic aromatic compounds by causing the monocyclic aromatic compounds to desorb therefrom through depressurization of the inside of the second vessel 420 using the second depressurization pump P3.

Examples of the monocyclic aromatic compounds include benzene, toluene, ethylbenzene, xylene, aniline, nitrobenzene, cumene, benzenesulfonic acid, chlorobenzene, benzoic acid, trinitrotoluene, and styrene, and one or more of these may be included.

The second adsorbent 42a may be constituted of the same kinds of materials as are listed for the first adsorbent 41a. Preferably, the second adsorbent 42a is constituted of activated carbon. This is because activated carbon particularly exhibits excellent adsorption and desorption performance for monocyclic aromatic compounds.

The average particle diameter of the second adsorbent 42a is preferably 1 mm to 20 mm, more preferably 2 mm to 15 mm, and even more preferably 3 mm to 10 mm.

The BET specific surface area of the second adsorbent 42a is preferably 100 m²/g to 2500 m²/g, more preferably 300 m²/g to 2000 m²/g, and even more preferably 500 m²/g to 1500 m²/g.

The average pore size of the second adsorbent 42a is preferably 1 Å to 300 Å, more preferably 5 Å to 250 Å, and even more preferably 10 Å to 200 Å.

Within these ranges, a sufficiently large contact area between the second adsorbent 42a and the exhaust gas is ensured.

When the exhaust gas further includes halogen compounds and cyanide compounds, the second separation section 42 is preferably configured to also adsorb and separate the halogen compounds and the cyanide compounds. This prevents adverse effects on the growth of gas-assimilating bacteria.

Examples of the halogen compounds include hydrogen chloride, hydrogen fluoride, chlorine, fluorine, and bromine. Examples of the cyanide compounds include hydrogen cyanide and nitriles.

When the material, average particle diameter, BET specific surface area, average pore size, density, temperature resistance, and shape of the second adsorbent 42a are appropriately selected, the second adsorbent 42a is capable of adsorbing not only monocyclic aromatic compounds but also the halogen compounds and the cyanide compounds.

At least one of the first vessel 410 and the second vessel 420 is preferably connected to a purge gas supply unit 43 configured to supply purge gas on the side opposite to the side to which the exhaust gas is supplied. In the present embodiment, the purge gas supply unit 43 is connected to the upper port 412 of the first vessel 410 via a gas line GL41 and to the upper port 422 of the second vessel 420 via a gas line GL42 branched from the gas line GL41.

A valve V7 is provided at an intermediate position of the gas line GL41, and a valve V8 is provided at an intermediate position of the gas line GL42.

With the foregoing configuration, the first adsorbent 41a, the first protective agent 41b1, the second protective agent 41b2, and the second adsorbent 42a are washed and regenerated by depressurizing the interior of the first vessel 410 by operation of the first depressurization pump P2 and depressurizing the interior of the second vessel 420 by operation of the second depressurization pump P3 while purge gas is supplied to the first vessel 410 and the second vessel 420.

The purge gas is preferably a gas unlikely to be adsorbed by or to react with the first adsorbent 41a, the first protective agent 41b1, the second protective agent 41b2, and the second adsorbent 42a. Specific examples of the purge gas include nitrogen gas and noble gases such as argon gas and helium gas. These gases may be used alone or in combination of two or more thereof.

Thermometers TE are connected to the first vessel 410 and the second vessel 420, respectively. This enables measurement of the temperatures inside the first vessel 410 and the second vessel 420.

Pressure gauges PT are connected to the lower port 411 and the upper port 412 of the first vessel 410 and to the lower port 421 and the upper port 422 of the second vessel 420, respectively. This enables measurement of the gas pressures supplied to and discharged from the first vessel 410 and the second vessel 420.

A hygrometer AE is connected at an intermediate portion of gas line GL12. This enables measurement of the humidity of the exhaust gas passing through gas line GL12.

The exhaust gas from which the monocyclic aromatic compounds have been separated by the second separation section 42 is supplied to the bioreactor 2.

The concentration of carbon dioxide in the exhaust gas supplied to the bioreactor 2 is preferably 0.1 vol% to 30 vol%, more preferably 0.3 vol% to 25 vol%, even more preferably 0.5 vol% to 20 vol%, particularly preferably 0.8 vol% to 15 vol%, and most preferably 1 vol% to 10 vol%.

The concentration of carbon monoxide in the exhaust gas supplied to the bioreactor 2 is preferably 10 vol% to 80 vol%, more preferably 15 vol% to 50 vol%, and even more preferably 20 vol% to 45 vol%.

The concentration of hydrogen in the exhaust gas supplied to the bioreactor 2 is preferably 1 vol% to 45 vol%, more preferably 5 vol% to 35 vol%, and even more preferably 10 vol% to 30 vol%.

The concentration of nitrogen in the exhaust gas supplied to the bioreactor 2 is preferably 30 vol% or less, more preferably 1 vol% to 25 vol%, and even more preferably 5 vol% to 20 vol%.

According to the foregoing configuration, since the concentration of carbon monoxide is increased by passing the exhaust gas through the pretreatment section, the organic substance can be efficiently produced in the bioreactor 2.

In the bioreactor 2, an organic substance is produced from the carbon monoxide included in the supplied exhaust gas by action of the gas-assimilating bacteria.

The purification device 6 is connected to the bioreactor 2 via the liquid line LL. The purification device 6 purifies the organic substance contained in the organic-substance-containing solution.

Examples of the purification device 6 include: a distillation device; a processing device including a pervaporation membrane; a processing device including a zeolite dehydration membrane; a processing device including an organic membrane; a processing device configured to remove low-boiling substances having boiling points lower than that of the organic substance; a processing device configured to remove high-boiling substances having boiling points higher than that of the organic substance; and a processing device including an ion-exchange membrane. These devices may be used alone or in combination of two or more thereof.

The temperature inside the distillation device during distillation of the organic substance (particularly ethanol) is not particularly limited but is preferably 100 °C or lower, more preferably 70-95 °C. When the temperature is within the foregoing range, separation between the desired organic substance and other components-that is, distillation (purification) of the organic substance-is performed more reliably.

The pressure inside the distillation device during distillation of the organic substance may be atmospheric pressure, but is preferably below atmospheric pressure (reduced-pressure distillation), more preferably 60-95 kPaA. When the pressure is within the foregoing range, the separation efficiency for the organic substance is improved, leading to an improved purity of the organic substance.

The concentration (purity) of the organic substance in the purified product is preferably 90 wt% or higher, more preferably 99 wt% or higher, and even more preferably 99.5 wt% or higher.

Organic substances obtained in this manner include, for example, monoalcohols such as methanol and ethanol; diols such as 2,3-butanediol; acetic acid; lactic acid; isoprene; and butadiene. Preferably, the organic substance is a monoalcohol or diol having 1 to 4 carbon atoms, more preferably ethanol.

The organic substance may be used, for example, as a raw material for resin and rubber materials and as various solvents, disinfectants, and fuels. High-concentration ethanol may be used as fuel ethanol blended into gasoline and the like; it may also be used, for example, as a raw material for cosmetics, beverages, chemicals, and fuels (including jet fuel), and as a food additive, and thus has very high versatility.

Next, a method of using the production system 100 of the first embodiment (organic substance production method) is described.
[1] First, the exhaust gas (raw-material gas including carbon monoxide, carbon dioxide, monocyclic aromatic compounds, and other gas components) discharged from the gasification furnace 10 is supplied to the filter device 3.

At this time, fine solids (for example, tar and soot) present in the exhaust gas are removed.

[2] Next, the valves V1, V2, V3, and V4 are opened, the valves V5, V6, V7, and V8 are closed, and the air supply pump P1 is operated. This causes the exhaust gas discharged from the filter device 3 to pass through the first separation section 41 (the first vessel 410).

At this time, polycyclic aromatic compounds and tar are adsorbed by the second protective agent 41b2, and moisture and sulfur compounds are adsorbed by the first protective agent 41b1. Removal of these substances suitably prevents or suppresses time-dependent deterioration in the performance of the first adsorbent 41a. Thereafter, carbon dioxide is adsorbed by the first adsorbent 41a.

The pressure of the exhaust gas delivered by the air supply pump P1 is preferably 0.1 MPaG or lower, more preferably 0.01 MPaG to 0.05 MPaG.

[3] Thereafter, the exhaust gas discharged from the first separation section 41 and separated from carbon dioxide and the like passes through the second separation section 42 (the second vessel 420).

At this time, monocyclic aromatic compounds, halogen compounds, and cyanide compounds are adsorbed by the second adsorbent 42a.

[4] Next, the exhaust gas discharged from the second separation section 42 passes through the catalyst device 5.

At this time, oxygen and acetylene are removed from the exhaust gas.

[5] Thereafter, the exhaust gas separated from monocyclic aromatic compounds and the like is supplied to the bioreactor 2. In the bioreactor 2, an organic substance is produced from carbon monoxide present in the supplied exhaust gas through the action of gas-assimilating bacteria. As a result, an organic-substance-containing solution that contains an organic substance is obtained.

Here, the temperature for producing the organic substance from carbon monoxide in the bioreactor 2 (culture temperature of the gas-assimilating bacteria) is not particularly limited but is preferably 25 °C to 50 °C.

[6] Next, the organic-substance-containing solution obtained in the bioreactor 2 is supplied to the purification device 6 via the liquid line LL. In the purification device 6, the organic substance in the organic-substance-containing solution is purified to obtain a product containing the organic substance at a high concentration.

[7] When a predetermined time has elapsed and the performance of the second protective agent 41b2, the first protective agent 41b1, and the first adsorbent 41a has deteriorated, the valves V1 and V2 are closed, the valve V5 is opened, and the first depressurization pump P2 is operated. This causes carbon dioxide and the like to separate from the first adsorbent 41a and the like in the first vessel 410. The carbon dioxide and the like are separated from the first adsorbent 41a by using a pressure change. Consequently, an off-gas containing carbon dioxide and the like can be collected.

The pressure inside the first vessel 410 resulting from depressurization by operation of the first depressurization pump P2 is preferably at or below -10 kPaG, more preferably at or below -30 kPaG, although not particularly limited. Use of the above-described first adsorbent 41a, first protective agent 41b1, and second protective agent 41b2 allows carbon dioxide and the like to be sufficiently desorbed without increasing the degree of depressurization, thereby reducing energy consumption.

At this time, the valve V7 may be opened to supply purge gas from the purge gas supply unit 43 to the first vessel 410. This increases the efficiency of desorption of carbon dioxide and the like from the first adsorbent 41a and the like, and also enables washing of the first adsorbent 41a and the like.

By optimizing the material, average particle diameter, BET specific surface area, average pore size, density, temperature resistance, and shape of the first adsorbent 41a, the first protective agent 41b1, and the second protective agent 41b2, the degree of depressurization by the first depressurization pump P2 can be reduced, or the first depressurization pump P2 may be omitted.

[8] Similarly, when a predetermined time has elapsed and the performance of the second adsorbent 42a has deteriorated, the valves V3 and V4 are closed, the valve V6 is opened, and the second depressurization pump P3 is operated. This causes monocyclic aromatic compounds and the like to separate from the second adsorbent 42a and the like in the second vessel 420. The monocyclic aromatic compounds and the like are separated from the second adsorbent 42a by using a pressure change. Consequently, an off-gas containing monocyclic aromatic compounds and the like can be collected.

The pressure inside the second vessel 420 resulting from depressurization by operation of the second depressurization pump P3 is not particularly limited but is preferably at or below -10 kPaG, more preferably at or below -30 kPaG. By using the above-described second adsorbent 42a, monocyclic aromatic compounds and the like can be sufficiently desorbed without increasing the degree of depressurization, thereby reducing energy consumption.

At this time, the valve V8 may be opened to supply purge gas from the purge gas supply unit 43 to the second vessel 420. This increases the efficiency of desorption of monocyclic aromatic compounds and the like from the second adsorbent 42a and enables washing of the second adsorbent 42a.

By optimizing the material, average particle diameter, BET specific surface area, average pore size, density, temperature resistance, and shape, and the like of the second adsorbent 42a, the degree of depressurization by the second depressurization pump P3 may be reduced, or the second depressurization pump P3 may be omitted.

According to the foregoing configuration, there is no need to heat the purge gas supplied to the first vessel 410 and the second vessel 420, which also contributes to a reduction in thermal energy consumption.

Further, by appropriately setting the material, average particle diameter, BET specific surface area, average pore size, packing amount, and order of arrangement of the first adsorbent 41a, the second adsorbent 42a, the first protective agent 41b1, and the second protective agent 41b2, the separation device 4 can be made compact, and the diameters of the connected piping and the pump capacity can be set accordingly, thereby enabling the production system 100 to be made compact.

### <Second Embodiment>

Next, an organic substance production system according to the second embodiment is described below.

In the following, the organic substance production system according to the second embodiment is described mainly with a focus on differences from the organic substance production system of the first embodiment, and description of similar matters is omitted.

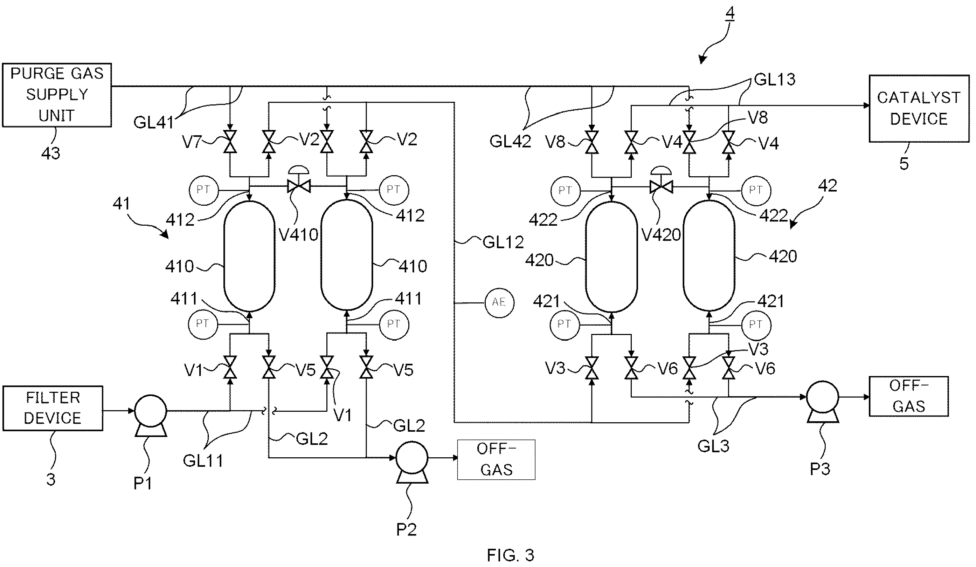

FIG. 3 is a schematic diagram showing the configuration of the organic substance production system according to the second embodiment.

In the production system 100 of the second embodiment, the configuration of the separation device 4 differs, and the other configurations are similar to those of the production system 100 of the first embodiment.

As shown in FIG. 3, the production system 100 includes a first separation section 41 having two first vessels 410 and a second separation section 42 having two second vessels 420.

The production system 100 of this second embodiment provides the same operations and effects as the production system 100 of the first embodiment.

In particular, in the second embodiment, since two first vessels 410 and two second vessels 420 are provided, while purge gas is supplied to one of the two first vessels 410, exhaust gas can be supplied to the other; similarly, while purge gas is supplied to one of the two second vessels 420, exhaust gas can be supplied to the other. Accordingly, continuous operation is possible without stopping the production system 100.

In the second embodiment, a valve V410 that connects the upper ports 412 of the two first vessels 410 and a valve V420 that connects the upper ports 422 of the two second vessels 420 are provided. These valves V410 and V420 are pressure-equalizing valves for reducing rapid pressure fluctuations and loss of gas when gas adsorption/desorption steps are repeated between the two first vessels 410 and between the two second vessels 420.

### <Third Embodiment>

Next, an organic substance production system according to the third embodiment is described below.

In the following, the organic substance production system according to the third embodiment is described mainly with a focus on differences from the organic substance production systems of the first and second embodiments, and description of similar matters is omitted.

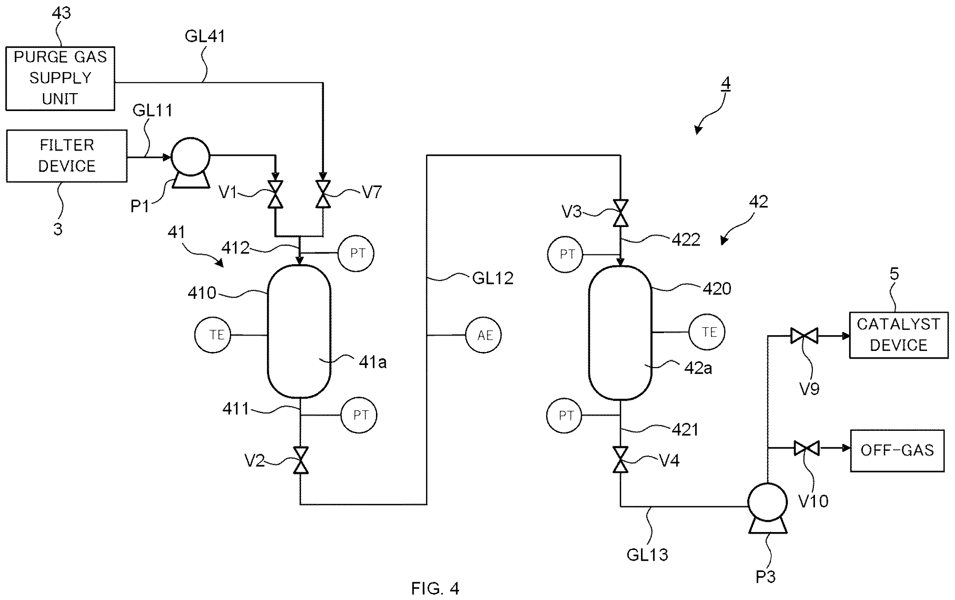

FIG. 4 is a schematic diagram showing the configuration of the organic substance production system according to the third embodiment.

In the production system 100 of the third embodiment, the configuration of the separation device 4 differs, and the other configurations are similar to those of the production system 100 of the first embodiment.

As shown in FIG. 4, the production system 100 is configured such that the first vessel 410 (first separation section 41) and the second vessel 420 (second separation section 42) are connected in series, and a single second depressurization pump P3 provided at an intermediate portion of gas line GL13 depressurizes the interior of the first vessel (first housing section) 410 and the interior of the second vessel (second housing section) 420. Thus, in the third embodiment, one second depressurization pump P3 constitutes the depressurization mechanism.

The production system 100 of the present third embodiment also provides the same operations and effects as those of the above-described production system 100 of the first embodiment.

In particular, in the production system 100 of the third embodiment, by adjusting the switching timings of valves V1 and V7 and the switching timings of valves V9 and V10, exhaust gas from which unwanted gas components have been removed and off-gas containing unwanted gas components can be separated and recovered using a single second depressurization pump P3.

### <Fourth Embodiment>

Next, an organic substance production system according to a fourth embodiment is described.

In the following, the organic substance production system according to the fourth embodiment is described mainly with a focus on differences from the organic substance production systems of the first to third embodiments, and description of similar matters is omitted.

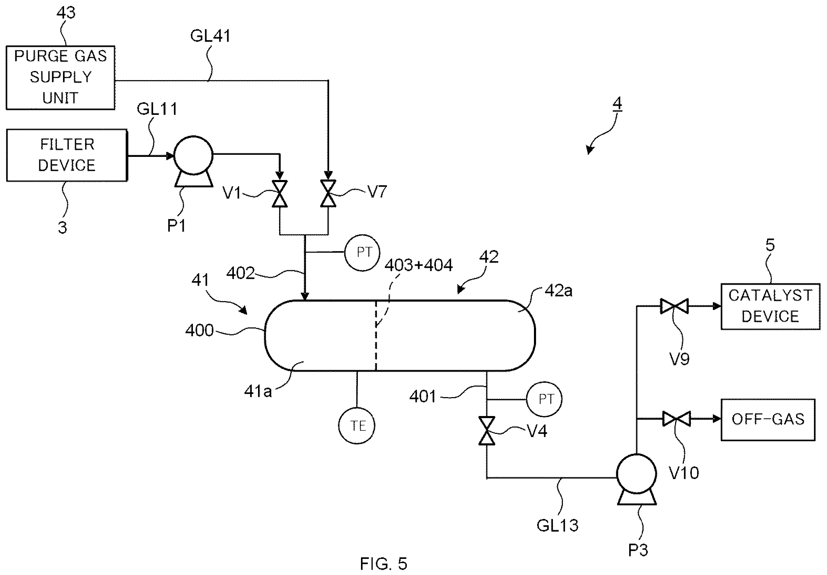

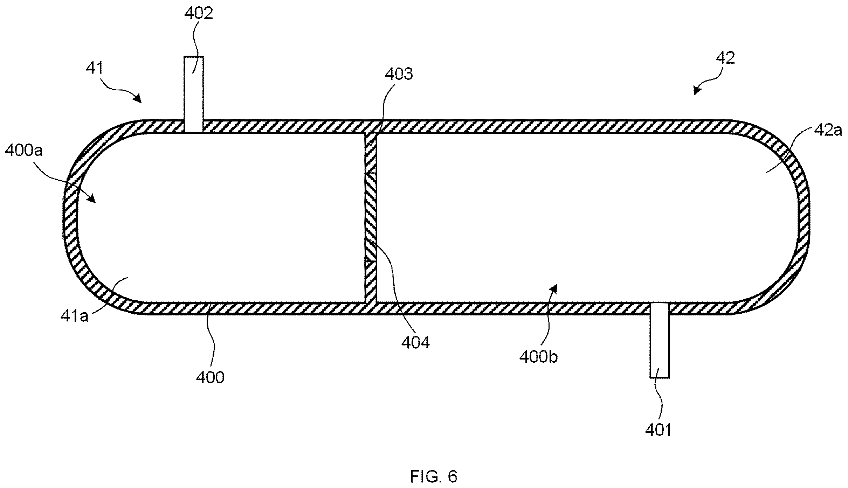

FIG. 5 is a schematic diagram showing the configuration of the organic substance production system according to the fourth embodiment. FIG. 6 is a schematic diagram illustrating the configuration of the separation device in the organic substance production system according to the fourth embodiment.

In the production system 100 of the fourth embodiment, the configuration of the separation device 4 differs, and the other configurations are similar to those of the production system 100 of the third embodiment.

The production system 100 shown in FIG. 5 includes a single vessel 400. As shown in FIG. 6, a partition wall 403 is disposed at an intermediate position in the longitudinal direction inside the vessel 400, thereby partitioning the interior into a left first accommodating space (first housing section) 400a and a right second accommodating space (second housing section) 400b.

In addition, a first adsorbent 41a is disposed in the first accommodating space 400a, and a second adsorbent 42a is disposed in the second accommodating space 400b.

Accordingly, in this embodiment, the left region of the vessel 400 constitutes the first separation section 41, and the right region constitutes the second separation section 42.

In addition, a valve element 404 is provided at a central portion of the partition wall 403. The valve element 404 opens in response to a pressure difference between the first accommodating space 400a and the second accommodating space 400b, thereby placing the two spaces 400a and 400b in communication with each other.

The valve element 404 preferably has high heat resistance and can be configured, for example, as a check valve, a duckbill valve, or the like. Alternatively, a filter may be disposed in place of the valve element 404.

The interior of the vessel 400 is configured such that it can be depressurized by a single second depressurization pump P3 provided at an intermediate portion of gas line GL13. That is, also in the fourth embodiment, a single second depressurization pump P3 constitutes the depressurization mechanism.

The production system 100 of this fourth embodiment also provides the same operations and effects as those of the above-described production systems 100 of the first to third embodiments.

In particular, in the production system 100 of the fourth embodiment, by adjusting the switching timings of valves V1 and V7 and the switching timings of valves V9 and V10, exhaust gas from which unwanted gas components have been removed and off-gas containing unwanted gas components can be separated and recovered using a single second depressurization pump P3.

According to the organic substance production system, the organic substance production apparatus, and the organic substance production method described above, an organic substance is producible while reducing the thermal energy used in the pretreatment. In addition, by appropriately setting the configuration of the adsorbent, a reduction in energy consumption is also achievable.

In the above embodiments, use of a pump as the depressurization mechanism has been described as one example; however, the depressurization mechanism is not limited to a pump. For example, by cooling any location of the production system 100 (production apparatus 1) to reduce pressure, a cooling device capable of depressurizing the interior of the first housing section and the interior of the second housing section may constitute the depressurization mechanism. In each example according to the present disclosure, the state before depressurization was 50 kPaG to 100 kPaG, and the state after depressurization was between -10 kPaG and -100 kPaG. Note that kPaG denotes a unit of gauge pressure, and gauge pressure is a value obtained by subtracting atmospheric pressure from absolute pressure.

Further, the disclosure may also be provided in the following aspects.
(1) An organic substance production system, comprising: a gas generation unit configured to generate a raw material gas including at least carbon monoxide, carbon dioxide, and monocyclic aromatic compounds; a first separation section including a first adsorbent capable of adsorbing at least the carbon dioxide in the raw material gas and a first housing section in which the first adsorbent is disposed, the first separation section being configured to separate the carbon dioxide from the first adsorbent by depressurizing the inside of the first housing section; a second separation section including a second adsorbent capable of adsorbing at least the monocyclic aromatic compounds in the raw material gas and a second housing section in which the second adsorbent is disposed, the second separation section being configured to separate the monocyclic aromatic compounds from the second adsorbent by depressurizing the inside of the second housing section; a depressurization mechanism configured to depressurize the inside of the first housing section and the inside of the second housing section; and a bioreactor configured to receive the raw material gas from which the carbon dioxide and the monocyclic aromatic compounds have been separated and to produce an organic substance from the carbon monoxide included in the supplied raw material gas by an action of gas-assimilating bacteria.
(2) The organic substance production system according to (1), wherein the first adsorbent is composed of zeolite.
(3) The organic substance production system according to (1) or (2), wherein: the raw material gas further includes moisture, sulfur compounds, polycyclic aromatic compounds, and tar; and the first separation section is configured to adsorb and separate the moisture, the sulfur compounds, the polycyclic aromatic compounds, and the tar.
(4) The organic substance production system according to (3), wherein a protective agent capable of adsorbing the moisture, the sulfur compounds, the polycyclic aromatic compounds, and the tar is further disposed in the first housing section to protect the first adsorbent.
(5) The organic substance production system according to (4), wherein the protective agent includes a first protective agent capable of adsorbing the moisture and the sulfur compounds and a second protective agent capable of adsorbing the polycyclic aromatic compounds and the tar.
(6) The organic substance production system according to (5), wherein the second protective agent, the first protective agent, and the first adsorbent are disposed in the first housing section in this order from a side to which the raw material gas is supplied.
(7) The organic substance production system according to (5) or (6), wherein the first protective agent is composed of zeolite, and the second protective agent is composed of activated carbon.
(8) The organic substance production system according to (7), wherein the first adsorbent is composed of zeolite having a larger pore size than the zeolite composing the first protective agent.
(9) The organic substance production system according to any one of (1) to (8), wherein: the depressurization mechanism includes a first depressurization section capable of depressurizing the inside of the first housing section, and the first depressurization section is connected to a side of the first housing section to which the raw material gas is supplied.
(10) The organic substance production system according to any one of (1) to (9), wherein: the raw material gas further includes halogen compounds and cyanide compounds, and the second separation section is configured to adsorb and separate the halogen compounds and the cyanide compounds.
(11) The organic substance production system according to (10), wherein: the depressurization mechanism includes a second depressurization section configured to depressurize the inside of the second housing section, and the second depressurization section is connected to a side of the second housing section to which the raw material gas is supplied.
(12) The organic substance production system according to any one of (1) to (11), wherein the second adsorbent is composed of activated carbon.
(13) The organic substance production system according to any one of (1) to (12), wherein at least one of the first housing section and the second housing section is connected to a purge gas supply unit configured to supply a purge gas on a side opposite to a side to which the raw material gas is supplied.
(14) An organic substance production apparatus configured to be used in connection with a gas generation unit configured to generate a raw material gas including at least carbon monoxide, carbon dioxide, and monocyclic aromatic compounds, comprising: a first separation section including a first adsorbent capable of adsorbing at least the carbon dioxide in the raw material gas and a first housing section in which the first adsorbent is disposed, the first separation section being configured to separate the carbon dioxide from the first adsorbent by depressurizing the inside of the first housing section; a second separation section including a second adsorbent capable of adsorbing at least the monocyclic aromatic compounds in the raw material gas and a second housing section in which the second adsorbent is disposed, the second separation section being configured to separate the monocyclic aromatic compounds from the second adsorbent by depressurizing the inside of the second housing section; a depressurization mechanism configured to depressurize the inside of the first housing section and the inside of the second housing section; and a bioreactor configured to receive the raw material gas from which the carbon dioxide and the monocyclic aromatic compounds have been separated and to produce an organic substance from the carbon monoxide included in the supplied raw material gas by an action of gas-assimilating bacteria.
(15) A method for producing an organic substance, comprising: adsorbing carbon dioxide from a raw material gas containing at least carbon monoxide, the carbon dioxide and monocyclic aromatic compounds using a first adsorbent, and separating the carbon dioxide from the first adsorbent using a pressure change; adsorbing the monocyclic aromatic compounds from the raw material gas using a second adsorbent and separating the monocyclic aromatic compounds from the second adsorbent using a pressure change; and supplying the raw material gas from which the carbon dioxide and the monocyclic aromatic compounds have been separated and producing an organic substance from the carbon monoxide included in the supplied raw material gas by an action of gas-assimilating bacteria.

The present disclosure is not limited thereto.

As described above, various embodiments of the present invention have been explained; these are presented by way of example and are not intended to limit the scope of the invention in any way. The novel embodiments may be implemented in various other forms, and various omissions, substitutions, and modifications may be made without departing from the spirit of the invention. Such embodiments and their modifications are included in the scope and spirit of the invention and are also encompassed within the scope of the invention set forth in the claims and equivalents thereof.

For example, in the organic substance production system, the organic substance production apparatus, and the organic substance production method according to the present invention, any desired configurations of the above first through fourth embodiments may be combined.

The organic substance production system and the organic substance production apparatus according to the present invention may each include any additional configurations relative to the above embodiments, may be replaced with any configurations that provide similar functions, and may omit some configurations.

The organic substance production method according to the present invention may include any additional steps relative to the above embodiments, may be replaced with any steps that provide similar functions, and may omit some steps.

### [code description]

100: Production system for organic substances
10: Gasification furnace
1: Production system for organic apparatus
2: Bioreactor
3: Filter device
4: Separation device
400: Vessel
400a: First accommodating space (first housing section)
400b: Second accommodating space (second housing section)
401: Lower port
402: Upper port
403: Partition wall
404: Valve element
41: First separation section
410: First container (first housing section)
411: Lower port
412: Upper port
41a: First adsorbent
41b: Protective agent
41b1: First protective agent
41b2: Second protective agent
42: Second separation section
420: Second container (second housing section)
421: Lower port
422: Upper port
42a: Second adsorbent
43: Purge gas supply unit
5: Catalyst device
6: Purification device
GL1: Gas line
GL11: Gas line
GL12: Gas line
GL13: Gas line
GL2: Gas Line
GL3: Gas Line
GL41: Gas line
GL42: gas line
LL: Liquid line
P1: Air supply pump
P2: First depressurization pump
P3: Second depressurization pump
V1: Valve
V2: Valve
V3: Valve
V4: Valve
V5: Valve
V6: Valve
V7: Valve
V8: Valve
V9: Valve
V10: Valve
V410: Valve
V420: Valve
AE: Hygrometer
PT: Pressure gauge
TE: Thermometer

## Claims

1. An organic substance production system, comprising:
a gas generation unit configured to generate a raw material gas including at least carbon monoxide, carbon dioxide, and monocyclic aromatic compounds;
a first separation section including a first adsorbent capable of adsorbing at least the carbon dioxide in the raw material gas and a first housing section in which the first adsorbent is disposed, the first separation section being configured to separate the carbon dioxide from the first adsorbent by depressurizing the inside of the first housing section;
a second separation section including a second adsorbent capable of adsorbing at least the monocyclic aromatic compounds in the raw material gas and a second housing section in which the second adsorbent is disposed, the second separation section being configured to separate the monocyclic aromatic compounds from the second adsorbent by depressurizing the inside of the second housing section;
a depressurization mechanism configured to depressurize the inside of the first housing section and the inside of the second housing section; and
a bioreactor configured to receive the raw material gas from which the carbon dioxide and the monocyclic aromatic compounds have been separated and to produce an organic substance from the carbon monoxide included in the supplied raw material gas by an action of gas-utilizing bacteria.

2. The organic substance production system according to claim 1, wherein
the first adsorbent is composed of zeolite.

3. The organic substance production system according to claim 1 or 2, wherein:
the raw material gas further includes moisture, sulfur compounds, polycyclic aromatic compounds, and tar; and
the first separation section is configured to adsorb and separate the moisture, the sulfur compounds, the polycyclic aromatic compounds, and the tar.

4. The organic substance production system according to claim 3, wherein
a protective agent capable of adsorbing the moisture, the sulfur compounds, the polycyclic aromatic compounds, and the tar is further disposed in the first housing section to protect the first adsorbent.

5. The organic substance production system according to claim 4, wherein
the protective agent includes a first protective agent capable of adsorbing the moisture and the sulfur compounds and a second protective agent capable of adsorbing the polycyclic aromatic compounds and the tar.

6. The organic substance production system according to claim 5, wherein
the second protective agent, the first protective agent, and the first adsorbent are disposed in the first housing section in this order from a side to which the raw material gas is supplied.

7. The organic substance production system according to claim 5 or 6, wherein
the first protective agent is composed of zeolite, and the second protective agent is composed of activated carbon.

8. The organic substance production system according to claim 7, wherein
the first adsorbent is composed of zeolite having a larger pore size than the zeolite composing the first protective agent.

9. The organic substance production system according to any one of claims 1 to 8, wherein:
the depressurization mechanism includes a first depressurization section capable of depressurizing the inside of the first housing section, and
the first depressurization section is connected to a side of the first housing section to which the raw material gas is supplied.

10. The organic substance production system according to any one of claims 1 to 9, wherein:
the raw material gas further includes halogen compounds and cyanide compounds, and
the second separation section is configured to adsorb and separate the halogen compounds and the cyanide compounds.

11. The organic substance production system according to claim 10, wherein:
the depressurization mechanism includes a second depressurization section configured to depressurize the inside of the second housing section, and
the second depressurization section is connected to a side of the second housing section to which the raw material gas is supplied.

12. The organic substance production system according to any one of claims 1 to 11, wherein
the second adsorbent is composed of activated carbon.

13. The organic substance production system according to any one of claims 1 to 12, wherein
at least one of the first housing section and the second housing section is connected to a purge gas supply unit configured to supply a purge gas on a side opposite to a side to which the raw material gas is supplied.

14. An organic substance production apparatus configured to be used in connection with a gas generation unit configured to generate a raw material gas including at least carbon monoxide, carbon dioxide, and monocyclic aromatic compounds, comprising:
a first separation section including a first adsorbent capable of adsorbing at least the carbon dioxide in the raw material gas and a first housing section in which the first adsorbent is disposed, the first separation section being configured to separate the carbon dioxide from the first adsorbent by depressurizing the inside of the first housing section;
a second separation section including a second adsorbent capable of adsorbing at least the monocyclic aromatic compounds in the raw material gas and a second housing section in which the second adsorbent is disposed, the second separation section being configured to separate the monocyclic aromatic compounds from the second adsorbent by depressurizing the inside of the second housing section;
a depressurization mechanism configured to depressurize the inside of the first housing section and the inside of the second housing section; and
a bioreactor configured to receive the raw material gas from which the carbon dioxide and the monocyclic aromatic compounds have been separated and to produce an organic substance from the carbon monoxide included in the supplied raw material gas by an action of gas-utilizing bacteria.

15. A method for producing an organic substance, comprising:
adsorbing carbon dioxide from a raw material gas containing at least carbon monoxide, the carbon dioxide and monocyclic aromatic compounds using a first adsorbent, and separating the carbon dioxide from the first adsorbent using a pressure change;
adsorbing the monocyclic aromatic compounds from the raw material gas using a second adsorbent and separating the monocyclic aromatic compounds from the second adsorbent using a pressure change; and
supplying the raw material gas from which the carbon dioxide and the monocyclic aromatic compounds have been separated and producing an organic substance from the carbon monoxide included in the supplied raw material gas by an action of gas-utilizing bacteria.
